# EUROPEAN PATENT APPLICATION

(11) **EP 1 724 573 A1**
(43) Date of publication of application: **22.11.2006**
(21) Application number: 06252634.8
(22) Date of filing: 19.05.2006
(51) Int. Cl.: G01N 27/403, G01N 33/543, G01N 33/483

(54) **Electrochemical method and apparatus for assaying or detecting biological material**

(30) Priority: 20.05.2005 JP 2005147512
(71) Applicant: SEIKO EPSON CORPORATION, Shinjuku-ku, Tokyo 163-0811 (JP)
(72) Inventor: Yokokawa, Shinobu, Seiko Epson Corporation, Nagano-ken 399-0785 (JP); Takiguchi Hiroshi, Seiko Epson Corporation, Nagano-ken 399-0785 (JP); Fukushima Hitoshi, Seiko Epson Corporation, Nagano-ken 399-0785 (JP)
(74) Representative: Cloughley, Peter Andrew

(57) **Abstract**

A method for detecting or assaying a target material in a sample solution, including: a first process for forming a metal oxide thin film containing a target material model on an electrode substrate; a second process for forming, on the metal oxide thin film, a recess into which the target material is able to engage, by removing the target material model from the metal oxide thin film; a third process for having the sample solution, into which a redox reactive molecule is added, contact the metal oxide thin film in which the recess is formed; and a fourth process for electrochemically detecting a transition of electron exchange with the redox reactive molecule in the vicinity of the electrode substrate surface, before and after the third process.

## Description

The present invention relates to a method for electrochemically detecting or assaying a target material in a sample solution, and to an electrode substrate, a detecting device, and a kit used for the same.

Various biosensors for identifying the existence of a target molecule in a sample solution or for checking the concentration of a sample solution are developed extensively. These common biosensors function by immobilizing molecules with a specific affinity to target molecules to a solid phase surface as probe molecules and thereafter by contacting the sample solution and the solid phase surface, thereby detecting and assaying the molecules that bond to the probe molecule. Examples of biomolecular combinations having a specific affinity with each other include: enzyme and substrate, antigen and antibody, nucleic acid and nucleic acid, and receptor and ligand. Detecting components that detect such intermolecular interaction suggested for the above-mentioned biosensors include: oxygen electrode, hydrogen peroxide solution, ion electrode, ion-selective field effect transistor (ISFET), fiber optics, thermistor and the like. Moreover, QCM (Quartz Crystal Microbalance) and surface plasmon resonance devices and the like, which can detect mass transition in the level of nanogram order, have also been in use recently.

In producing such biosensors, the selection of methods for immobilizing probe molecules to a solid surface is very critical. The above-mentioned intermolecular interaction occurs at a specific bonding moiety or a functional group in each molecule. Therefore, the immobilization must be done in a state, where the bonding moiety and the functional group are recognized by the target molecule and are able to interact. Hence, many methods that select functional groups and spacers according to the variation of the solid phase surface, so that binding capacities are maintained, and bond the molecules to the solid phase surface via those functional groups and spacers, have been suggested.

In JP-A-11-90214 for instance, a method is suggested, as one of the methods for manufacturing thin films containing biogenic substance, to deploy a solution or an aqueous dispersion of materials such as protein, nucleic acid, sugar, lipid, and virus, and transcribe it to the solid surface. With this method, since proteins etc. are arrayed on the hydrogel surface, an ultrathin film containing these biomolecules is formed on the solid surface, by having the smooth solid substrate contact the gel surface and transcribing the substance on to the solid surface.

In JP-A-2004-351608 for instance, a system using an amorphous metal oxide which utilizes compounds that include an organic/metal alkoxide group is also suggested. The surface sol-gel approach (refer to *"*Expected Materials for the Future (Mirai Zairyo)', Vol.3 Issue 8, page 20 to 27), on which the above technique is based, produces an ultrathin film of metal oxides by chemically adsorbing the solid substrate that has the hydroxy group on its surface with the metal alkoxide compound, and hydrolyzing the metal alkoxide compound. In JP-A-2001-351608, a method is disclosed where a mold is formed on the solid base with lithography, and a metal oxide thin film is deposited on the formed mold, forming a metal oxide nanostructure by removing the formed mold.

When immobilizing biomolecules on the solid surface, it is extremely difficult to control the location and direction of the biomolecules so that the binding capacity thereof is maintained even through the spacer. Further, the immobilized biomolecules may change their structure in the liquid phase, or may be decomposed, hence loosing their binding capacity. This involves a problem of not being able to conduct a detection even if a target material exists in a sample, since the material does not bond with probe molecules.

The advantage of the invention is to provide a technique that allows the improvement of the image quality of electrophoretic devices.

According to a first aspect of the invention, a method for detecting or assaying a target material in a sample solution, including: a first process for forming a metal oxide thin film containing a target material model on an electrode substrate; a second process for forming, on the metal oxide thin film, a recess into which the target material is able to engage, by removing the target material model from the metal oxide thin film; a third process for having the sample solution, into which a redox reactive molecule is added, contact the metal oxide thin film in which the recess is formed; and a fourth process for electrochemically detecting a transition of electron exchange with the redox reactive molecule in the vicinity of the electrode substrate surface, before and after the third process.

The "target material model" used in accordance with the above and following aspects of the invention indicates a material that has an identical or a similar shape as that of the target material to be detected or assayed, or, preferably, the same material as that of the target material. Hence, by removing the target material model from the metal oxide thin film that contains the target material model, recesses with identical or approximately the same shape as that of the target material are formed. Thereafter, by having the sample solution contact the metal oxide thin film, the target material specifically engages itself to the recess, as seen in the relationship of receptor and ligand. Consequently, the state of electron exchange with the redox reactive molecule in the vicinity of the electrode substrate surface changes, allowing an easy confirmation of the existence of the target material in a high sensitivity, by electrochemically detecting this change. If a quantitative measurement of the state of the electron exchange is achieved, then it also allows an concentration estimation of the target material in the sample solution.

The sample solution in the above aspect of the invention indicates the target solution for detection and assay of the target material, and the target material may either be contained in a resolved state or a dispersed state.

The "redox reactive molecule" used in accordance with the above aspect of the invention may include various compounds, and is not specifically limited, as long as it is reversibly redox reactive. Examples of such compounds include: potassium ferricyanide (K3Fe(CN)6) and a group of compounds with ferrocene structure. Iron included in these compounds is in a divalent ion state, and thereafter changes to a tervalent ion state after releasing an electron (oxidized). By this reversible redox reaction, a current, proportional to the amount of compound, can be extracted by applying a voltage.

It is preferable that, in the method of detection or assay of the target material, the target material be selected from a group including an organic molecule, a biomolecule, a cell, a microorganism, and a virus. The above structure allows an easy and highly sensitive detection of the material with a highly persistence metal oxide thin film mold, whereas the material has been conventionally detected based on a biological specificity.

It is preferable that, in the above-mentioned second process, the metal oxide thin film be formed in such a thickness so that only one layer of the target material model is included therein. This is because, if the metal oxide thin film is too thick, the target material model is buried deep inside the metal oxide thin film, and after the removal of the target material model, the recess is not exposed, thus the target material can not engage with the recess.

It is preferable that, in the above-mentioned second process, the metal oxide thin film is formed with a surface sol-gel approach using a metal alkoxide compound. With the surface sol-gel approach, the film thickness of the metal oxide thin film can be controlled by nanometer, and the film thickness where the target material is contained only in a single layer can easily be formed.

It is preferable that, in the above-mentioned second process, the removal of the target material model be conducted with a processing selected from a group including: oxygen plasma processing, ozone oxidation processing, elution processing, and firing processing. The above structure allows the removal of only the target material model in the metal oxide thin film, and the recess, into which the target material can specifically engage, is formed in the place where the target material model used to be before removal.

It is preferable that, the detection in the above-mentioned fourth process of the target material model be conducted with a measurement system selected from a group including: cyclic voltammetry, potentiostatic, galvanostatic, and impedance measurements. The above measurement systems allow the electrochemical detection of the electron exchange of the redox reactive molecule in the vicinity of the electrode surface, thereby detecting and assaying the target material.

It is preferable that, in the method of detection or assay of the target material, in the above-mentioned fourth process, the determination be made that the target material exists in the sample solution, if the electron exchange in the vicinity of the substrate surface, with the redox reactive molecule resolved in the sample solution, declines. Since the engagement of the target material with the recess of the metal oxide thin film blocks the migration site of the redox reactive molecule, if the target material exists in the sample solution, then the amount of electron exchange is reduced in the vicinity of the substrate surface.

According to a second aspect of the invention, an electrode substrate is provided where the electrode substrate includes a thin film formed of metal oxide on the surface of the electrode substrate, wherein, on the thin film, a recess to which the target material is able to engage is formed. With the above structure, if the target material exists in the sample solution, it engages into the recess of the metal oxide thin film, thereby changing the state of the electron exchange in the vicinity of the electrode substrate surface. By detecting this change, the existence of the target material in the sample solution is detected and assayed.

Here, it is preferable that the above-mentioned recess be formed by forming a metal oxide thin film containing a target material model on the surface of the electrode substrate, and removing the target material model from the metal oxide thin film. With this structure, the target material engages specifically with the recess.

According to a third aspect of the invention, a detecting device is provided, where this device for detecting or assaying a target material in a sample solution includes the aforementioned electrode substrate, a counter electrode that faces the electrode substrate, and a reference electrode. The above structure allows an easy and highly sensitive detection of the electron exchange status change at the surface of the electrode substrate.

It is preferable that this detecting device further include a detecting circuit to which the electrode substrate, the counter electrode, and the reference electrode are independently and electrically connected. The above structure allows an easy and highly sensitive detection of the electron exchange status change at the surface of the electrode substrate with the detecting device.

According to a fourth aspect of the invention, a kit for detecting or assaying a target material in a sample solution includes: an electrode substrate; a material containing a metal oxide compound for forming a thin film on the electrode substrate; and a redox reactive molecule which performs electron exchange in the vicinity of the electrode substrate. The above kit allows the user to form the metal oxide thin film that contains an arbitrary target material model, thereafter to remove it, and to produce the electrode substrate for detecting the desired target material. Using this electrode substrate allows the appropriate detection and assay of the target material that is in accordance with the above aspects of the invention.

According to a fifth aspect of the invention, a kit for detecting or assaying a target material includes: the aforementioned detecting device according to the third aspect of the invention; and a redox reactive molecule which performs electron exchange in the vicinity of the electrode substrate. The above structure allows an easy and highly sensitive detection of the prescribed target material, without the user having to prepare a reagent necessary for target material and for the measurement.

The invention will be described by way of example only and with reference to the accompanying drawings, wherein like numbers reference like elements, and wherein:

Figs.1A to 1D illustrate a flow indicating an outline of detection and assay of a target material according to the embodiment of the invention;

Fig.2 is a schematic top view drawing of a device for detecting and assaying the target material according to the embodiment of the invention;

Fig.3 is a schematic top view drawing of a device for detecting and assaying the target material according to the embodiment of the invention;

Fig.4 is a schematic oblique drawing of a system for detecting and assaying the target material according to the embodiment of the invention; and

Fig.5 is a measurement result of a detection of oligopeptide as the target material, in accordance with the method in the embodiment of the invention.

The embodiments of the invention will now be described with reference to the drawings. The following embodiments are examples for describing the aspects of the invention, and the invention shall not be limited to these embodiments. The invention may be embodied with various kinds of modifications as long as the modifications do not depart from the scope of the invention.

First Embodiment

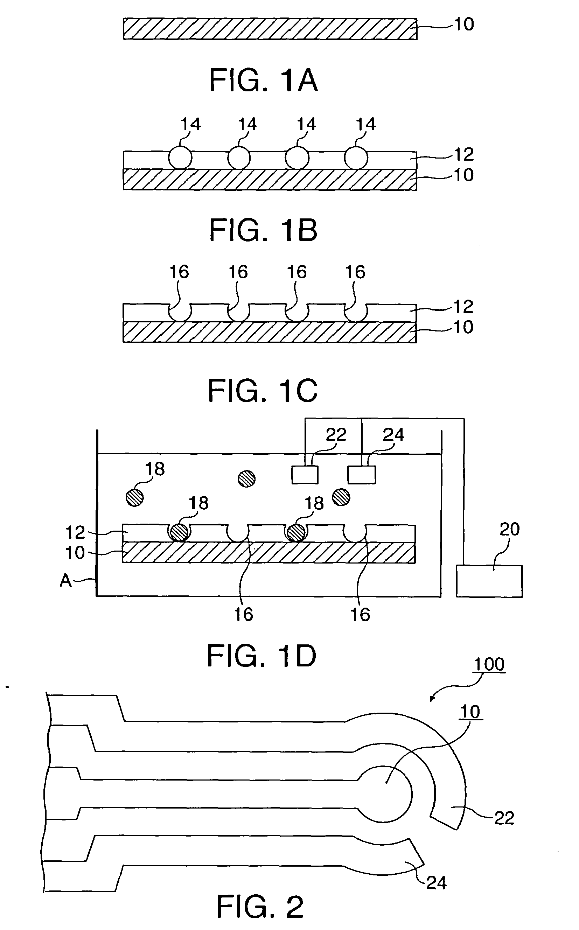

Figs.1A to 1D illustrate a flow indicating an outline of detection or assay of a target material according to the embodiment of the invention.

First Process: Metal Oxide Thin Film Formation

A metal oxide thin film 12 that contains a target material model 14 is formed on a electrode substrate 10, as shown in Figs.1A and 1B. The formation of the ultrathin film is desirably performed by a surface sol-gel approach that uses a metal alkoxide compound.

Here, the surface sol-gel approach indicates a system of chemically adsorbing a hydroxy group etc. on the electrode substrate 10 with the metal alkoxide compound, and thereafter hydrolyzing them, thereby forming a monomolecular metal oxide film immobilized on the substrate surface in a covalent bond and on the multilayer of the metal oxide film. More specifically, the electrode substrate that has a functional group such as a hydroxy group that is reactive to the metal alkoxide is dipped into a metal alkoxide solution for few minutes. Subsequently, the substrate is washed with a prescribed organic solvent so that the physically adsorbed metal alkoxide is removed, and thereafter, by dipping the substrate into ion-exchange water, the hydrolysis of the metal alkoxide and the polycondensation of the surface is prompted. The new hydroxy group produced on hydrolysis in the outermost layer may again be utilized for a chemical adsorption of the metal alkoxide compound. Hence, by repeating the adsorption and hydrolysis, the multilayer of the metal oxide with the thickness of nanometer level for each layer may be produced.

The metal oxide thin film 12 containing a target material model 14 may be produced with the surface sol-gel approach, by alternately performing a surface adsorption of, for instance, the target material model 14 and the metal alkoxide compound, so that the multilayer of the metal oxide that contains the target material model 14 between the layers is formed. Alternatively, by letting the target material model 14 that has an active hydroxy group react with the metal alkoxide compound in advance, in order to form the complex of the two, and by consecutively adsorbing the complex on the solid surface by the surface sol-gel approach, the metal oxide thin film containing organic molecules or biomolecules may be formed. The target material model 14 may be adsorbed on the surface of the electrode substrate 10 electrostatically, and thereafter, the metal oxide thin film 12 may also be deposited so as to fill the space between the target material models 14 using the surface sol-gel approach. With this method of depositing the metal oxide thin film 12, the film may be formed in the thickness that includes only a single layer in which the target material model 14 is included, since the film thickness thereof can be controlled by nanometer.

Here, the phrase "the film thickness where the target material model 14 is contained only in a single layer" means that if the target material model 14 is suborbicular for instance, the film thickness of the metal oxide thin film 12 is approximately the same as or thinner than the diameter thereof. If the target material model 14 is linear shaped, the film thickness of the metal oxide thin film 12 is approximately the same or less than the length of the target material model 14. If the film thickness of the metal oxide thin film 12 is too thick, the target material model 14 does not get exposed to the surface; hence, the recess into which the target material can engage is not formed even when the target material model 14 is removed. However, this film thickness control is not necessary, when, for instance, using the target material model 14 with smaller relative gravity than the metal alkoxide, and the metal oxide thin film 12 may be formed in a way that the target material model 14 is exposed to the surface.

The functional group on the substrate surface may also be one that is active to the metal alkoxide, such as carboxyl, and is not limited to the hydroxy group. Moreover, a variation of the metal oxide thin film is not specifically limited, as long as it is synthesized from the metal alkoxide compounds; hence various metal oxide ultrathin films may be produced, depending on the variations of the metal alkoxide compound.

Further, with the surface sol-gel approach, the thin film is formed based on the adsorption of the metal alkoxide compound in the solution. Hence, a film with even thickness may be formed, not being dependent on the shape of the substrate.

In the surface sol-gel approach, by changing the mobility of the redox reactive molecules toward the electrode, using an adjustment of the composition of the metal alkoxide as well as an introduction of a porous structure, the insulation of the metal oxide to be formed is controlled easily. Hence the adjustment of the insulation that suits the electrochemical detection is achieved. The composition of the metal alkoxide includes not only pure alkoxide, but also alkyl substituents, as well as compounds where groups such as vinyl, phenyl, and isocyanate are introduced, mixed in an arbitrary ratio.

The target material model will now be described in further detail. As described above, the target material model is a material that has an identical or similar shape as that of the target material to be detected or assayed, or, preferably, the same material as that of the target material. The target material is selected from a substance such as an organic molecule, a biomolecule, a cell, and a microorganism, so that the appropriate target material is selected. Here, the biomolecule may be protein, nucleic acid, sugar, or lipid, and is not limited as long as it is a biogenic molecule.

Second Process: Target Material Model Removal

Subsequent to the first process, the target material model 14 is removed from the metal oxide thin film 12, and the recess 16 into which the target material can engage is formed, as shown in Fig.1C. The method for removing the target material model 14 includes oxygen plasma processing, ozone oxidation processing, elution processing, and firing processing. However, particularly the oxygen plasma processing is preferable. With the oxygen plasma processing, only the organic molecules are removed and sintered, and the recess 16 having the shape of the target material model is formed accurately in the metal oxide thin film 12. The elution processing using alkaline solution such as ammonia water also allows a clear removal of the target material model.

Third Process: Contact between the Sample Solution and Metal Oxide Thin Film

Subsequent to the second process, as shown in Fig.1D, the electrode substrate 10 is dipped into the sample solution A, in order to have the metal oxide thin film 12 contact the sample solution A. This allows the target material 18 to engage into the recess 16, if the target material exists in the sample solution.

In the present embodiment, the entire electrode substrate 10 is dipped into the sample solution. However, droplets containing the sample solution may also be deposited on the metal oxide thin film 12.

Fourth Process: Electrochemical Detection

Subsequent to the third process, as shown in Fig.1D, the electron exchange in the vicinity of the surface of the electrode substrate 10 is electrochemically detected by a device 20. The electrochemical detection may be conducted with measurements such as cyclic voltammetry (CV), potentiostatic, galvanostatic, and impedance measurements. In the embodiment, as shown in Fig.1D, the electrode substrate 10, a counter electrode 22 that faces it, and a reference electrode 24 are connected to a detection circuit in the device 20, thereby detecting the current of the vicinity of the electrode substrate 10 surface.

Hereafter, the measurement principle is described in outline. As shown in Fig.1D, in the sample solution into which the electrode substrate 10 is dipped, the redox reactive molecules that assist the mobility of electrons such as ferrocene are dissolved. Here, by applying a suitable voltage between the electrode substrate 10 and the counter electrode 22, the redox reactive molecules mobilize electrons. The metal oxide thin film 12 adjusts its composition as described above, and by introducing the porous structure, the redox reactive molecules conducts favorable electron exchange with the electrode surface. However, if the target material 18 engages itself to the recess 16, it results in blocking the electron exchange of this location, and the reduction of electron exchange (amount of current) is detected.

Therefore, if the amount of electron exchange declines, the engagement of the target material 18 to the recess 16 and the existence of the target material 18 in the sample solution A are confirmed. The quantitative measurement of the decline of current allows a quantitative estimation of the target material 18 in the sample solution. If the amount of current does not change, then there is no engagement of the target material to the recess 16, which leads to the potential conclusion that the target material 18 does not exist in the sample solution A.

The recess may be reused, if the target material 18 is removed after the measurement, from the metal oxide thin film 12 with the oxygen plasma processing, since the recess into which the target material can engage is formed again.

As described above, with this method of detection and assay according to the embodiment of the invention, by forming the recess 16 into which the target material 18 can engage in the metal oxide thin film 12, the detection of the existence of the target material in the sample solution and the assay thereof is achieved.

Since the above method does not utilize the specific bonding among biomolecules, there is no need to immobilize anything in order to maintain the bonding moiety of molecules or the binding capacity of a specific functional group. The biomolecules used as the target material model are ultimately removed, and the moiety that captures the target material is formed only with the metal oxide. Therefore, the bonding property does not change with a time lapse, excelling in persistence, and allowing the reuse as described above, by re-sintering.

Moreover, there is a benefit in cost, since the detection and assay of the target material is performed by measuring the electron-transferring substance being blocked by the target material, saving the labeling of a marker material.

Second Embodiment

The electrode substrate according to the aspects of the invention and a device for target material detection and assay that is provided with the electrode substrate, will now be described as a second embodiment of the present invention.

On the surface of the electrode substrate, a thin film formed with metal oxide is formed, and on the thin film, the recess moiety into which the target material can engage is formed. In Fig.1C, this electrode substrate is illustrated as the electrode substrate 10 on which the metal oxide thin film 12 is formed. On the metal oxide thin film 12, the recess 16 into which the target material can engage is formed. Such electrode substrate 10 excels in persistence and stability, compared to microarrays where the biogenic substance is immobilized, and can be distributed independently. The method for manufacturing the electrode substrate 10 is omitted since it is described in the section of the first embodiment.

As described, the electrode substrate 10 may either be: an independent structure, where the counter electrode and the reference electrode are dipped into the sample solution A, and thereafter the electrochemical measurement is performed; or an united structure being at one with the counter electrode 22 and the reference electrode 24, constituting a detection and assay device.

An example of such device is shown in Fig.2.

Fig.2 illustrates a conceptual top view drawing of a detecting device 100 that includes the electrode substrate 10, the counter electrode 22 that faces the electrode substrate 10, and the reference electrode 24. The detecting device 100 shown in Fig.2 indicates only the major electrode structure in the example. The exemplary material that forms the counter electrode 22 used in this embodiment may be, but not limited to, platinum. The reference electrode 24 serves as a reference electrode for the electrode substrate 10 and the counter electrode 22, and the exemplary material thereof may be, but not limited to, silver chloride.

The metal oxide thin film, on which recesses are formed so that the target material can engage thereto, is formed on the surface of the electrode substrate 10. If, for instance, the sample solution is dropped so as to cover the counter electrode 22, reference electrode 24, and the electrode substrate 10, then the electron exchange occurs in the vicinity of the surface of the electrode substrate 10. By electrically connecting the counter electrode 22, reference electrode 24, and the electrode substrate 10 independently to a detecting circuit 120, the generated current may be measured with the detecting circuit 120. The exemplary component that constitutes the detecting circuit 120 used in this embodiment may be, but not limited to, thin film transistor and the like. The current measurement may be performed electrochemically. Examples of a measurement system include: cyclic voltammetry, differential pluse voltammetry, potentiostatic, galvanostatic, and impedance measurements.

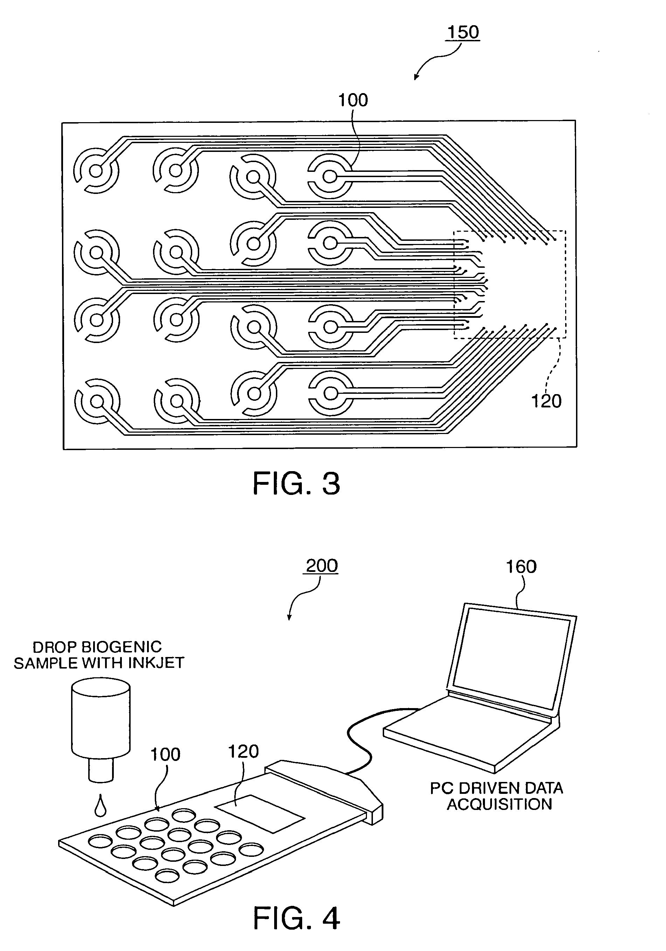

Fig.3 illustrates a conceptual top view drawing of a device 150 that includes a plurality of detecting devices 100 according to the embodiment of the invention, and the detecting circuit 120 that is electrically connected to each of the plurality of detecting devices 100. In the electrical connection between the detecting circuit 120 and the detecting device 100, the electrode substrate 10, the counter electrode 22, and the reference electrode 24 are independently connected to the detecting circuit 120. If thin film transistors are used in the detecting circuit 120, the electrode substrate 10 may be connected to the drain of the thin film transistor, and the current value measured in the electrode substrate 10 may be received and amplified.

As shown in Fig.3, in this device, the simultaneous target material detection and assay can be performed with one or more samples, by either having the individual detecting device 100 contact a single sample or a variation of samples, or by forming the metal oxide thin film that contains recesses into which the different target materials can engage. Moreover, even when using the same sample, a measurement may be performed in a wider measurable range of sample concentrations, by letting the individual detecting device 100 contact the sample, where the amount of the target material model introduced to the detecting device 100 is modified so that the number of recesses 16 is adjusted. The material that forms the circuit that connects the detecting circuit 120 and each of the detecting devices 100 may be, but not limited to, silver wiring and the like.

Fig.4 illustrates a conceptual top view drawing of a system 200, where the device 150 (shown in Fig.3) that is connected to a personal computer (hereafter simply referred to as "PC") 160, is driven by the PC. Here, the device 150 is disposable, covered with, for instance, a low-cost material such as plastic and the like. The exemplary component that constitutes the plastic substrate used in this embodiment may be, but not limited to, acrylic resin, polycarbonate resin, etc. By making the device disposable, the contamination is prevented when used for infinitesimal amount of target material detection. Connecting the device 150 to the PC allows a PC-driven detection, where the information is transmitted through the thin film transistor (the detecting circuit 120), the information being obtained in the thin film transistor, via an interface such as USB. The information obtained from the thin film transistor may also be transmitted to the PC via a wireless communication, by installing a radio frequency (RF) tag connected to the thin film transistor in the device 150. The detection of the sample may also be performed, by having the droplets of the sample solution contact the electrode substrate 10 with methods such as microspotting or inkjet. Hence, an "in vitro", real-time detectable sensor system 200 is provided.

Third Embodiment

A kit for target material detection and assay, according to the fourth and fifth aspect of the invention, will now be described as a third embodiment of the present invention.

The kit in the third embodiment of the invention at least includes: an electrode substrate, a material that contains metal alkoxide compound for forming thin film on the electrode substrate, and redox reactive molecule. With such kit, users can select an arbitrary target material, and form the metal oxide thin film that contains the target material by using the metal alkoxide compound, on the surface of the electrode substrate. Thus the target material detection and assay may be appropriately performed using the above-mentioned electrode substrate. Here, it is suitable, as shown in Fig.1, that the electrode substrate is provided to the kit as the detecting device 100 including the counter electrode 22 and the reference electrode 24. It is also suitable that the system 200 including a plurality of such devices is provided to the kit.

The above-mentioned kit may also include a reagent used to form the metal oxide thin film, or a reagent used in the detection and assay process. Moreover, if required, it may also include a user manual, etc.

The present invention will now further be described using an example and a comparative example indicated below. However, the descriptions are for exemplary purpose only, and the invention shall not be limited to those specific examples. One skilled in art can embody the invention applying various modifications to the example below, and such modifications shall be included within the scope of the claims in this invention.

Example 1

Metal Oxide Thin Film Formation

An electrode used for the measurement were prepared by: vapor deposition of gold on a silicon substrate; ozone washing thereof; thereafter dripping the substrate to an 1mM mercapto propanoic acid ethanol solution for 12 hours; thereby introducing hydroxyl on the surface of the substrate. Subsequently, the substrate was washed with ethanol, sprayed with nitrogen gas, and was dried sufficiently. Since the surface of the substrate is electrified in negative, in the case where the target material electrified in positive is used, the substrate can be used as it is in order to adsorb the target material. However, in this example, oligopeptide electrified in negative was used as the target material and as the target material model; hence, the electrode substrate was dipped into a 1mg/mL polydiallyldimethylammonium chloride solution, and the surface thereof was set to positive. Here, three kinds of oligopeptide (Ala-Ala-Ala-Ala, Val-Val-Val-Val, and Ala-Ala-Val-Ala, where Ala represents alanine, and Val represents valine) are used.

Thereafter, the electrode substrate was dipped, for approximately 10 minutes, into a 0.1mg/mL oligopeptide phosphate buffer solution (pH7.2) as the target material model, and an electrostatic surface adsorption was performed thereon. Subsequently, this substrate was washed with ion-exchanged water, sprayed with nitrogen gas, and was dried sufficiently.

In the surface sol-gel approach, the substrate was dipped for 1 minute into a 100mM titanium isopropoxide (Ti(O-iPr)4) ethanol solution as the target material model, and an electrostatic surface adsorption was performed thereon. Subsequently, this substrate was washed with ion-exchanged water, sprayed with nitrogen gas, and was dried sufficiently. A multilayer film of titania is formed after repeating this surface sol-gel approach three times.

Subsequently, the metal oxide thin film that includes the above-mentioned oligopeptide as the target material model was placed in a sample chamber of an oxygen plasma generation device, and an oxygen plasma was directed to the thin film in a room temperature for 20 minutes under the condition of 176mTorr oxygen partial pressure and 10W of an high frequency output. Further, under the condition of 176mTorr oxygen partial pressure and 20W of the high frequency output, the target material model in the film was removed by directing plasma for 40 minutes in a room temperature. Here, the state of oligopeptide as the target material model, being removed by the oxygen plasma processing was evaluated with infrared reflection absorption measurement.

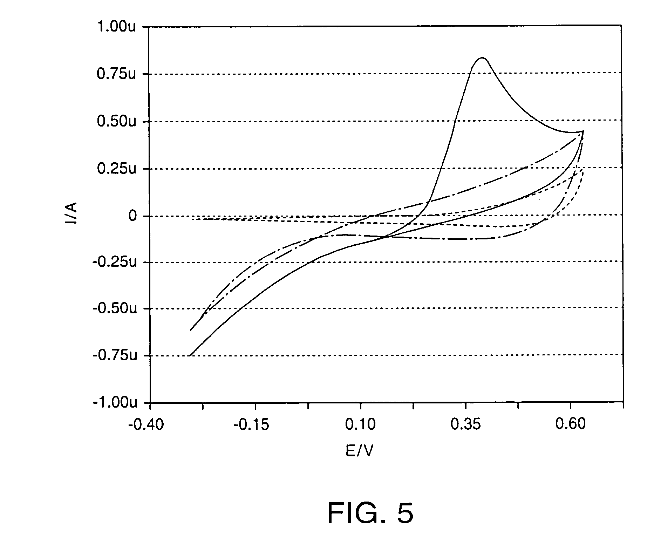

Thereafter, the formed substrate is dipped into a 10mL solution for electrochemical measurement (composition: 5mM potassium ferricyanide (K3Fe(CN)6), 20mM NaCl, 10mM phosphate buffer (ph7.2)), and the electrochemical measurements were conducted before and after adding 1mL of oligopeptide solution (0.1 to 10µg/mL inclusive). The results of the cyclic voltammetry measurement is shown in Fig.5. The electrochemical state of the target material (Ala-Ala-Ala-Ala), before engaging to the recesses, is indicated in a solid line. The state of oligopeptide with identical amino sequence as that of the target material model, after engaging to the recesses, is indicated in a dotted line. The state of oligopeptide, with partial difference in amino sequence (Ala-Ala-Val-Ala) compared to the target material model, after engaging to the recesses, is indicated in dashed line.

The complete engagement of oligopeptide that has identical amino sequence as that of the target material model inhibited the electron exchange in the vicinity of the electrode substrate surface, thereby significantly reducing the detected current originated from a redox reactive material. In the case where oligopeptide, with partial difference in amino sequence compared to that of the target material model, was engaged to the recess, there was a little reduction of current (not a significant reduction). Hence it is considered that the engagement took place, but incompletely.

The results confirms that with the method of target material detection and assay according to the invention allows recognition and detection of slight difference in amino sequence.

The aforegoing description has been given by way of example only and it will be appreciated by a person skilled in the art that modifications can be made without departing from the scope of the present invention.

## Claims

1. A method for detecting or assaying a target material in a sample solution, comprising:
a) forming a metal oxide thin film containing a target material model on an electrode substrate;
b) forming, on the metal oxide thin film, a recess into which the target material is able to engage, by removing the target material model from the metal oxide thin film;
c) making the sample solution, into which a redox reactive molecule is added, contact the metal oxide thin film in which the recess is formed; and
d) electrochemically detecting a transition of electron exchange with the redox reactive molecule in the vicinity of the electrode substrate surface, before and after the third process.

2. The method according to claim 1, wherein the target material is selected from a group including an organic molecule, a biomolecule, a cell, a microorganism, and a virus.

3. The method according to claim1, wherein, in the process b), the metal oxide thin film is formed in such a thickness so that only one layer of the target material model is included therein.

4. The method according to claim1, wherein, in the process b), the metal oxide thin film is formed with a surface sol-gel approach using a metal alkoxide compound.

5. The method according to claim1, wherein, in the process b), the removal of the target material model is conducted with a processing selected from a group including: oxygen plasma processing, ozone oxidation processing, elution processing, and firing processing.

6. The method according to claim1, wherein, the detection in the process d) of the target material model is conducted with a measurement system selected from a group including: cyclic voltammetry, potentiostatic, galvanostatic, and impedance measurements.

7. The method according to claim1, wherein, in the process d), in the case where the electron exchange in the vicinity of the substrate surface, with the redox reactive molecule resolved in the sample solution, declines, the determination is made that the target material exists in the sample solution.

8. An electrode substrate for detecting or assaying a target material in a sample solution, comprising:
a thin film formed of metal oxide on the surface of the electrode substrate; and
wherein, on the thin film, a recess to which the target material is able to engage is formed.

9. The electrode substrate according to claim 8, wherein the recess is formed by forming a metal oxide thin film containing a target material model on the surface of the electrode substrate, and removing the target material model from the metal oxide thin film.

10. A device for detecting or assaying a target material in a sample solution, comprising:
the electrode substrate according to claim 8;
a counter electrode that faces the electrode substrate; and
a reference electrode.

11. The device according to claim 10, further comprising a detecting circuit to which the electrode substrate, the counter electrode, and the reference electrode are independently and electrically connected.

12. A kit for detecting or assaying a target material in a sample solution, comprising:
an electrode substrate;
a material containing a metal oxide compound for forming a thin film on the electrode substrate; and
a redox reactive molecule which performs electron exchange in the vicinity of the electrode substrate.

13. A kit for detecting or assaying a target material, comprising:
the device according to claim 10; and
a redox reactive molecule which performs electron exchange in the vicinity of the electrode substrate.
